# EUROPEAN PATENT SPECIFICATION

(11) **EP 3 307 279 B1**
(45) Date of publication and mention of the grant of the patent: **04.12.2019**
(21) Application number: 16728041.1
(22) Date of filing: 10.06.2016
(51) Int. Cl.: A61K 31/727, A61P 37/06

(54) **METHOD FOR PREVENTING TRANSPLANT FAILURE IN A HOST.**
VERFAHREN ZUR VERHINDERUNG VON TRANSPLANTATVERSAGEN IN EINEM HOST
PROCÉDÉ PERMETTANT D'EMPÊCHER UNE DÉFAILLANCE DE TRANSPLANTATION CHEZ UN HÔTE

(30) Priority: 11.06.2015 EP 15171710
(43) Date of publication of application: 18.04.2018
(73) Proprietor: Universiteit Maastricht, 6211 LK Maastricht (NL); Academisch Ziekenhuis Maastricht, 6229 HX Maastricht (NL)
(72) Inventor: HEURN, VAN, Lodewijk Willem Ernest, 1105 AZ Amsterdam (NL); NICOLAES, Gerardus Anna Franciscus, 6265 BE Sint Geertruid (NL); REUTELINGSPERGER, Christiaan Peter Maria, 6211 KB Maastricht (NL); SMAALEN, VAN, Tim Christian, 6211 EC Maastricht (NL)
(74) Representative: Algemeen Octrooi- en Merkenbureau B.V.
(86) International application number: PCT/EP2016/063264
(87) International publication number: WO 2016/198578

(56) References cited:
- EP-A1- 2 545 924
- EP-A2- 0 251 134
- LODER O ET AL: "Suppression of Experimental Autoimmune Diseases and Prolongation of Allgraft Survival by Treatment of Animals with Low Doses of Heparins", JOURNAL OF CLINICAL INVESTIGATION, AMERICAN SOCIETY FOR CLINICAL INVESTIGATION, US, vol. 83, no. 3, 1 March 1989 (1989-03-01), pages 752-756, XP008109623, ISSN: 0021-9738, DOI: 10.1172/JCI113953
- GRADOWSKA L ET AL: "Low dose heparin: efficacious treatment for chronic renal allograft rejection", ARCHIVUM IMMUNOLOGIAE ET THERAPIAE EXPERIMENTALIS, BIRKHAEUSER VERLAG AG, CH, vol. 41, no. 2, 1 January 1993 (1993-01-01), pages 133-135, XP008178049, ISSN: 0004-069X
- STEVENS R B ET AL: "Administration of nonanticoagulant heparin inhibits the loss of glycosaminoglycans from xenogeneic cardiac grafts and prolongs graftsurvival", TRANSPLANTATION PROCEEDINGS, ELSEVIER INC, ORLANDO, FL; US, vol. 25, no. 1, 1 February 1993 (1993-02-01), page 382, XP008178048, ISSN: 0041-1345
- HOOK M ET AL: "Anticoagulant activity of heparin: Separation of high-activity and low-activity heparin species by affinity chromatography on immobilized antithrombin", FEBS LETTERS, ELSEVIER, AMSTERDAM, NL, vol. 66, no. 1, 1 July 1976 (1976-07-01), pages 90-93, XP025599609, ISSN: 0014-5793, DOI: 10.1016/0014-5793(76)80592-3 [retrieved on 1976-07-01]

## Description

### Field of the invention

This invention is in the field of medical treatment, in particular the invention provides a method for preventing, ameliorating or reducing graft failure after transplantation in a recipient.

### Background of the invention

Transplantation is a complex medical treatment that allows the transplantation of organs from a donor to a recipient whose organs failed to work sufficiently, improving the quality of life of the recipient, increasing life expectancy and saving lives.

Transplant failure is one of the greatest challenges to transplantation. After organ transplantation there is an inevitable response in the host and in the graft. This response may occur as result of trauma, associated with organ procurement, perfusion, preservation and surgery. This response may also involve specific recognition by the immune system of antigenic differences between donor and recipient (1). Together, these mechanisms may develop acute or after weeks, months or even years and lead to a destructive response that ultimately leads to loss of the graft. This process is also often referred to as transplant rejection.

Graft loss is a result of a variety of responses and begins with cell death processes, which if not stopped, will increase and cause a graft to fail. The traumatic injury leading to cell death and caused by organ procurement, preservation and ischemia/reperfusion injury cannot be entirely prevented. Therefore proper treatment strategies to diminish the negative effects are vital. Regrettably, there are not many options available.

Rejection is the outcome of the natural response of the immune system to a foreign substance, or antigen. This complex process is mainly T-lymphocyte mediated, although it involves serial interactions between foreign antigens, antibodies, T lymphocytes, macrophages, cytokines (also known as lymphokines or interleukins), adhesion molecules (ie, co- stimulatory molecules), and membrane proteins that enhance binding of T lymphocytes and B lymphocytes.

Immunosuppressive therapy is often performed to prevent and treat transplant rejection as well as to prolong transplant and patient survival. However, due to the potency of immunosupressive agents and inter- and intra-individual variability in pharmacokinetics, dose individualization is required to maintain adequate immunosuppression while minimizing adverse reactions. Poor water solubility and bioavailability contribute to the complexity of dosing immunosuppressive agents such as cyclosporine and sirolimus. Transplant rejection can be hyperacute (within the first hours after transplantation or during the early days) caused by preformed antibodies, acute (during the early days or months) caused by T-lymphocytes or chronic (months or even years later) mainly caused by antibodies.

There is an ongoing shortage of donor organs. This causes major health issues, because patients on the waiting list for an organ for transplantation are likely to die without organ transplantation. They are a heavy financial burden to the health care system due to large costs of their treatment (2-10).

Methods for increasing the success rate of transplantations are therefore urgently required.

### Summary of the invention

We found that a heparin with a decreased anti-coagulant activity, in particular a pentasaccharide-depleted heparin may be used to reduce, ameliorate or prevent transplant failure of a foreign organ in a recipient. Pentasaccharide-depleted heparin has a remarkably decreased anti-coagulant activity. Moreover, it showed a remarkable ability to neutralize histone-mediated cytotoxicity. This histone-mediated cytotoxicity is held responsible for graft failure in a transplantation. Hence, the invention relates to a pentasaccharide-depleted heparin for use in the treatment or prevention of transplant failure in the human or animal body.

### Detailed description of the invention

Heparin is a mixture of polysaccharide chains (Casu, B. (1989). "Structure of heparin and heparin fragments." Ann N Y Acad Sci 556: 1-17). The composition of the polysaccharide chains and their length varies. Chains with a so-called pentasaccharide domain bind strongly to anti-thrombin (AT), which is one of the major circulating anticoagulant proteins (Casu, B. et al. Biochem J 197(3) (1981) 599-609).

Heparin with a decreased anti-coagulant activity is known in the art and there are several methods known for its preparation. First, heparin may be depleted from its pentasaccharides by affinity chromatography, thereby obtaining non-anticoagulant heparin or heparin with a decreased anti-coagulant activity. Heparin may also be chemically treated in order to obtain heparin with a decreased anti-coagulant activity.

Pentasaccharide depleted heparin may be obtained from unfractionated heparin (UFH) by methods known in the art. In a preferred method, the pentasaccharide depleted heparin is obtained by affinity chromatography. Therein, UFH is passed through a column that contains immobilized AT. The molecules that contain the pentasaccharide sequence bind to the column, whereas other material passes. Unbound material is called Low Affinity Material (LAM), whereas material that does bind is called High Affinity Material (HAM). LAM is substantially reduced in pentasaccharides and subsequently in its anticoagulant activity, whereas HAM has full anticoagulant activity.

LAM may also be described as the pentasaccharide-depleted fraction of natural heparin.

The term pentasaccharide-depleted heparin in this context is used to refer to a fraction of heparin wherein the content of pentasaccharides is substantially reduced in comparison to commercially available heparin.

The term substantially reduced or decreased as used herein means reduced with at least 10%, such as 20% or 30%, more preferably 40 or 50%, even more preferred, more than 60% or 70% or 80% such as 90% or more than 98% such as more than 99% or even 100%. It is most preferred when the pentasaccharide depleted fraction does not contain any detectable pentasaccharides when tested for thrombin generation as described by Hemker et al., (2003) infra. Conversely, heparin with a decreased anti-coagulant activity means heparin with a reduced anti-coagulant activity, such as reduced with at least 10%, such as 20% or 30%, more preferably 40 or 50%, even more preferred, more than 60% or 70% or 80% such as 90% or more than 98% such as more than 99% or even 100%. It is most preferred when the heparin with a decreased anti-coagulant activity does not contain any detectable anti-coagulant activity when tested for thrombin generation as described by Hemker et al., (2003) infra.

In the experimental section it is described in detail how a pentasaccharide-depleted heparin may be obtained. It is called LAM therein, abbreviation of Low Affinity Material.

We found that by thus removing the anticoagulant heparin fraction from UFH yields a pentasaccharide depleted heparin that neutralizes histone-mediated cytotoxicity and that may advantageously be used to prevent transplant failure of donor organs and tissues such as heart, lung, liver, cornea, skin, uterus, kidney, pancreas and intestine.

The term transplant failure or graft failure as used herein means the failure of the transplanted organ to perform its normal function. This could mean that the transplanted organ fails to perform its function entirely or just in part. Transplant failure may also refer to a condition of the transplanted organ wherein its function is substantially reduced or decreased.

### Legend to the figures

Figure 1. Separation of UFH into LAM and HAM. 2 mg UFH was applied. The figure shows the optical density at 254 nm, wherein the first peak represents the collected LAM, the second peak contains the collected HAM. The second graph represents the conductivity of the eluted material. LAM was eluted with 1 ml/min and HAM with 4 ml/min.
Figure 2: Anticoagulant activity of various heparin preparations. Diagram showing that desulphation and pentasaccharide depletion both yield heparin fractions with a decreased anti-coagulant activity.
Figure 3: Neutralization of the cytotoxicity of histone H3. Diagram showing that histone H3 cytotoxicity is reduced in desulphated heparin fraction but fully intact in pentasaccharide-depleted heparin.

### Examples

### Example 1: Preparation of an AT-column.

The AT-column was prepared according to the package insert of a 5 ml HiTrap column (GE Healthcare®). After washing the isopropanol from the column -2.5 mg AT in 5 ml coupling buffer was applied to the column. Then, the described procedure to immobilize the protein to the column material and to wash the column was employed (according to the package insert). Finally the column was equilibrated with 140 mM NaCl, 20 mM Tris (pH 7.4).

### Example 2: Separation of UFH into LAM and HAM.

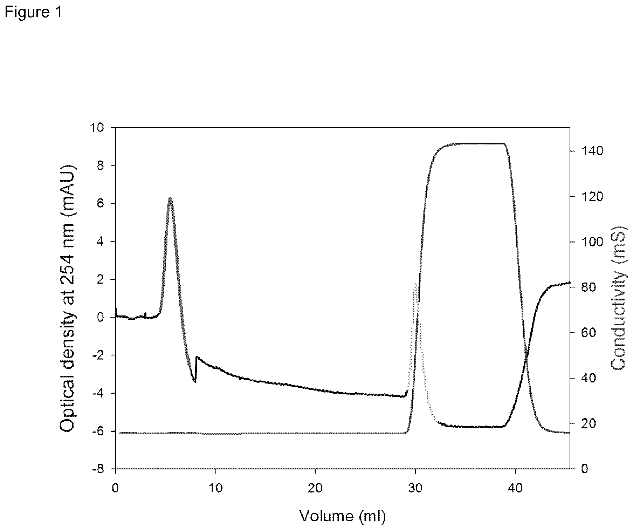

To the column was applied 2 mg unfractionated heparin. LAM was eluted with 140 mM NaCl, 20 mM Tris (pH 7.4) and HAM with 2 M NaCl, 20 mM Tris (pH 7.4). The last buffer was applied in a block gradient. In Fig. 1 an example of the elution pattern is shown.

To obtain a larger amount of LAM, the procedure described in Figure 1 was repeated several times.

To determine whether the LAM was free of HAM two tests were used. Firstly, collected HAM was reapplied to the AT-column and run as described above. No HAM-peak was found. Secondly the effect of LAM on thrombin generation was measured. The reaction mixture (120 µl) contained normal pooled plasma in a 1.5 × dilution, 3 µl LAM or buffer, 4 µM DOPL (60% DOPC, 20% DOPC and 20% DOPE), 5 pM tissue factor (Innovin), 100 mM CaCl2 and 417 µM ZGGR-AMC. The reaction was started with CaCl2 + ZGGR-AMC. Thrombin generation was measured as described by Hemker, H. C., P. Giesen, et al. (2003). "Calibrated automated thrombin generation measurement in clotting plasma." Pathophysiol Haemost Thromb 33(1): 4-15. Thrombin generation was not inhibited by the added 3 µl HAM.

The column fractions containing LAM were collected. The buffer was switched to ammonium bicarbonate (pH 7.8) with Sephadex G-25 medium and the fractions were lyophilized. Dried LAM was weighed and dissolved in phosphate buffered saline to reach the desired concentration.

### Example 3: Prevention of transplant failure

We made our observations in a kidney transplant model wherein we transplanted mouse kidneys. We determined the conditions under which the kidneys for transplantation had an increased chance of failure, and then conducted an experiment wherein 30 mice were given between 0.1 and 10 mg of LAM per mouse per injection. An appropriate mouse control group did not receive any treatment, another control group received comparable amounts of HAM or natural heparin.

It was observed that the group receiving LAM had a lower graft failure rate.

### Example 4: Anticoagulant activity of various heparin preparations

Unfractionated heparin (UFH) was pentasaccharide depleted by affinity chromatography on a column with immobilised antithrombin. The non-binding flow through contained the pentasaccharide depleted heparin (LAM).

Unfractionated heparin was desulfated according to the method described by Yuko Inoue and Kinzo Nagasawa, Carbohydrate Research (1976) 46: 87-95.

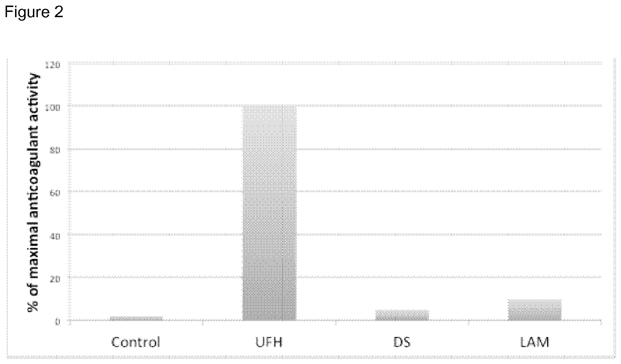

The desulfated heparin (DS), UFH and LAM were tested in the Endogenous Thrombin Potential (ETP) assay to determine their anticoagulant activity (Figure 2). We conclude that desulfation as well as depletion of pentasaccharide almost completely abolishes the anticoagulant activity of UFH, at least to an extent below 10% of the original activity of UFH.

### Example 5: Neutralization of the cytotoxicity of histone H3.

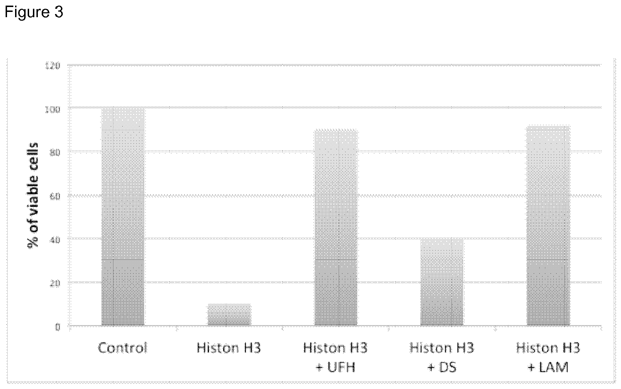

DS, UFH and LAM were tested in the Histon H3 cytotoxicity assay as described by Wildhagen et al. (Blood 2014; 123:1098-1101) to determine their protective activity towards the cytotoxicity of Histon H3 (Figure 3). It was observed that the protective effect towards histone H3 cytotoxicity was preserved in the LAM fraction (100% of the activity of unfractionated heparin (figure 3)), whereas desulphated heparin showed a significant reduction (more than 50%) in the protective effect in comparison with unfractionated heparin. We conclude that UFH and LAM neutralize the cytotoxic activity of Histon H3 to the same extent, whereas desulfation of heparin (DS) results in a loss of ability to neutralize the cytotoxic activity of Histon H3.

### References

1. Morris PJ, Knechtle SJ. Kidney Transplanatation: principles and practice. Sixth ed. Philadelphia: Saunders Elsevier; 2008.
2. Foundation E. Annual report 2013. 2013.
3. Colvin-Adams M, Smith JM, Heubner BM, Skeans MA, Edwards LB, Waller CD, et al. OPTN/SRTR 2013 Annual Data Report: heart. Am J Transplant. 2015;15 Suppl 2:1-28.
4. Israni AK, Zaun DA, Rosendale JD, Snyder JJ, Kasiske BL. OPTN/SRTR 2013 Annual Data Report: deceased organ donation. Am J Transplant. 2015;15 Suppl 2:1-13.
5. Kandaswamy R, Skeans MA, Gustafson SK, Carrico RJ, Tyler KH, Israni AK, et al. OPTN/SRTR 2013 Annual Data Report: pancreas. Am J Transplant. 2015;15 Suppl 2:1-20.
6. Kim WR, Lake JR, Smith JM, Skeans MA, Schladt DP, Edwards EB, et al. OPTN/SRTR 2013 Annual Data Report: liver. Am J Transplant. 2015;15 Suppl 2:1-28.
7. Matas AJ, Smith JM, Skeans MA, Thompson B, Gustafson SK, Stewart DE, et al. OPTN/SRTR 2013 Annual Data Report: kidney. Am J Transplant. 2015;15 Suppl 2:1-34.
8. Schnitzler MA, Skeans MA, Axelrod DA, Lentine KL, Tuttle-Newhall JE, Snyder JJ, et al. OPTN/SRTR 2013 Annual Data Report: economics. Am J Transplant. 2015;15 Suppl 2:1-24.
9. Smith JM, Skeans MA, Horslen SP, Edwards EB, Harper AM, Snyder JJ, et al. OPTN/SRTR 2013 Annual Data Report: intestine. Am J Transplant. 2015;15 Suppl 2:1-16.
10. Valapour M, Skeans MA, Heubner BM, Smith JM, Hertz MI, Edwards LB, et al. OPTN/SRTR 2013 Annual Data Report: lung. Am J Transplant. 2015;15 Suppl 2:1-28.

## Claims

1. Pentasaccharide-depleted heparin for use in preventing, ameliorating or reducing graft failure after transplantation in a recipient.

2. Pentasaccharide-depleted heparin for use according to claim 1 wherein the graft is an organ or a tissue.

3. Pentasaccharide-depleted heparin for use according to claim 2 wherein the organ is skin, heart, liver, lung, pancreas, intestine or kidney.

4. Pentasaccharide-depleted heparin for use according to any one of claims 1 - 3 wherein the pentasaccharide-depleted heparin is obtained by affinity chromatography.

5. Pentasaccharide-depleted heparin for use according claim 4 wherein the affinity chromatography was performed using immobilized anti-thrombin.

## Patentansprüche

1. Pentasaccharid-verarmtes Heparin zur Verwendung bei der Prävention, Verbesserung oder Reduzierung von Transplantatversagen nach der Transplantation bei einem Empfänger.

2. Pentasaccharid- verarmtes Heparin zur Verwendung nach Anspruch 1, wobei das Transplantat ein Organ oder ein Gewebe ist.

3. Pentasaccharid- verarmtes Heparin zur Verwendung nach Anspruch 2, wobei das Organ Haut, Herz, Leber, Lunge, Bauchspeicheldrüse, Darm oder Niere ist.

4. Pentasaccharid- verarmtes Heparin zur Verwendung nach einem der Ansprüche 1 - 3, wobei das mit Pentasaccharid verarmte Heparin durch Affinitätschromatographie erhalten wird.

5. Pentasaccharid- verarmtes Heparin zur Verwendung nach Anspruch 4, wobei die Affinitätschromatographie unter Verwendung von immobilisiertem Antithrombin durchgeführt wurde.

## Revendications

1. Héparine appauvrie en pentasaccharide pour une utilisation dans la prévention, l'amélioration ou la réduction de rejet de greffe après une transplantation chez un receveur.

2. Héparine appauvrie en pentasaccharide pour une utilisation selon la revendication 1, dans laquelle le greffon est un organe ou un tissu.

3. Héparine appauvrie en pentasaccharide pour une utilisation selon la revendication 2, dans laquelle l'organe est la peau, le cœur, le foie, le poumon, le pancréas, l'intestin ou le rein.

4. Héparine appauvrie en pentasaccharide pour une utilisation selon l'une quelconque des revendications 1 à 3, dans laquelle l'héparine appauvrie en pentasaccharide est obtenue par chromatographie d'affinité.

5. Héparine appauvrie en pentasaccharide pour une utilisation selon la revendication 4, dans laquelle la chromatographie d'affinité a été réalisée en utilisant l'antithrombine immobilisée.
